# EUROPEAN PATENT APPLICATION

(11) **EP 4 389 050 A1**
(43) Date of publication of application: **26.06.2024**
(21) Application number: 22307005.3
(22) Date of filing: 22.12.2022
(51) Int. Cl.: A61B 34/30, A61B 34/20, A61B 90/30, A61B 90/00

(54) **TRACKING ASSEMBLY AND ROBOTIC SYSTEM**

(71) Applicant: Ecential Robotics, 38610 Gieres (FR)
(72) Inventor: SIEFFERT, Jérôme, 38610 Gières (FR); VIDAL, Clément, 38610 Gières (FR)
(74) Representative: Regimbeau

(57) **Abstract**

An assembly (10) for tracking a position and orientation of a robotic arm (100) is provided. The assembly includes a hand grip (50) disposed between its proximal end (22) and its distal end (24). The assembly also includes a first exterior portion (42, 44) at the proximal end and a second exterior portion (46) at the distal end (24). Optical markers (30) are provided on those exterior portions (42, 44, 46) and distributed along a central axis (A) of the assembly. The hand grip (50) is smaller in size around the central axis (A) than the first exterior portion (42, 44).

## Description

### TECHNICAL FIELD

The present disclosure relates to a tracking assembly for tracking a position and orientation of a robotic arm and to a robotic system comprising such a tracking assembly. In particular, the present disclosure relates to a tracking assembly used for a surgical robotic system.

### TECHNICAL BACKGROUND

Surgical robotic systems are frequently used during surgical interventions in order to assist a surgeon in putting a tool guide in position relative to a surgical target with a desired orientation. Once the tool guide is brought in position, the surgeon inserts a surgical tool to the tool guide to maneuver the surgical tool at a given position with a given orientation. Alternatively, the tool guide can be replaced by a tool holder which is configured to keep a surgical tool in position relative to the robotic arm. Other examples of surgical robot systems are disclosed in US 2011/0290060, US 2020/0360092, US 2020/0155241 and US 2010/0168562.

Surgical robotic systems often rely on X-ray fluoroscopy to generate images of the inside of a patient's body in real time, thereby allowing real-time navigation of the surgical robotic arm during surgery. In particular, in orthopedic, spine and/or traumatological surgeries, X-ray fluoroscopy is employed to monitor fracture reductions to determine a precise position and orientation for the surgical tool to be inserted and/or for an implant to be placed relative to the surrounding bone segments.

In order to navigate the surgical robotic arms using X-ray fluoroscopy, a tracking assembly is provided onto the robotic arm to allow for real-time localization of the robotic arm and the corresponding end-effector relative to a surgical target. EP 3821843 discloses examples of such tracking assemblies that enable to track the position and orientation of a robotic arm and an end-effector in order to maintain its position and orientation relative to the surgical target.

Precise localization of the robotic arm and the corresponding end-effector is relevant to successful surgical interventions. Thus, a great deal of effort has been made in the field of surgical robots to improve precision in localizing the robotic arm and the end-effector.

There is also a need to reduce the overall size of a tracking assembly in order to improve manipulability and reachability of the surgical tool for the surgeon using the surgical robotic system. However, if the tracking assembly is relatively small, precise localization of the surgical tool tends to become more difficult.

### SUMMARY OF THE DISCLOSURE

One of the objectives of the present disclosure is to provide a tracking assembly that is reduced in size yet enables precise localization of a surgical tool attached to the tracking assembly.

According to one aspect of the present disclosure, there is provided an assembly for tracking a position and orientation of a robotic arm, the assembly comprising: a support body extending along a central axis and having a proximal end and a distal end opposite to the proximal end, the proximal end being configured to be attached to the robotic arm, the distal end being configured to be attached to a tool holder or a tool guide, the support body further comprising a hand grip extending around the central axis and configured to provide a grip to a user for controlling the robotic arm; and at least three optical markers disposed on the support body and distributed around the central axis, wherein the hand grip is disposed between the proximal end and the distal end, wherein the support body further comprises: at least one first exterior portion extending around the central axis and being disposed at the proximal end or between the hand grip and the proximal end, each first exterior portion being provided with at least one of the optical markers; and at least one second exterior portion extending around the central axis and disposed at the distal end or between the distal end and the hand grip, each second exterior portion being provided with at least one of the optical markers, and wherein the first exterior portion has an outermost edge which lies farther from the central axis in a direction perpendicular to the central axis than an outermost edge of the hand grip.

According to one aspect of the present disclosure, the support body may be a monolithic piece.

According to one aspect of the present disclosure, the assembly may further comprise at least one operational button for controlling the robotic arm, the at least one operational button being provided on the hand grip of the support body.

According to one aspect of the present disclosure, at least one of the first and second exterior portions may taper toward the distal end.

According to one aspect of the present disclosure, each of the first exterior portions may have an outermost edge at an equal distant or greater distance from the central axis in a direction perpendicular to the central axis than the outermost edge of the second exterior portion.

According to one aspect of the present disclosure, the support body may comprise two of the first exterior portions spaced apart from each other along the central axis and one of the second exterior portion.

According to one aspect of the present disclosure, each optical marker may be provided in a cavity formed on the corresponding exterior portion in such a way that the optical marker is substantially flush with a surface of the corresponding exterior portion.

According to one aspect of the present disclosure, the optical markers may be light emitting elements.

According to one aspect of the present disclosure, the assembly may further comprise a visible light emitting ring extending around the central axis.

According to one aspect of the present disclosure, the assembly may further comprise a storage device for storing data for tracking the position and orientation of the robotic arm.

According to one aspect of the present disclosure, the assembly may further comprise at least one electrical connector arranged in an interior of the support body, the at least one electrical connector being configured to engage with a corresponding electrical connector of the robotic arm.

According to one aspect of the present disclosure, the electrical connector may be electrically connected to the at least one operational button, the light emitting elements, the visible light emitting ring and the storage device.

According to one aspect of the present disclosure, there is provided a robotic system comprising: a multiple joints robotic arm; and the above-described assembly attached to a distal flange of the robotic arm.

According to one aspect of the present disclosure, the proximal end of the support body may be attached to the robotic arm in such a way that the distal flange of the robotic arm lies within an interior of the proximal end.

According to one aspect of the present disclosure, the robotic system may further comprise a sterile cover enclosing the robotic arm and the support body, wherein the sterile cover fits the first and second exterior portions of the support body.

### BRIEF DESCRIPTION OF THE FIGURES

Embodiments of the present disclosure will be described in further details with reference to the accompanying drawings, in which:
figure 1 is a perspective view showing a surgical robotic system including a tracking assembly according to one embodiment; and

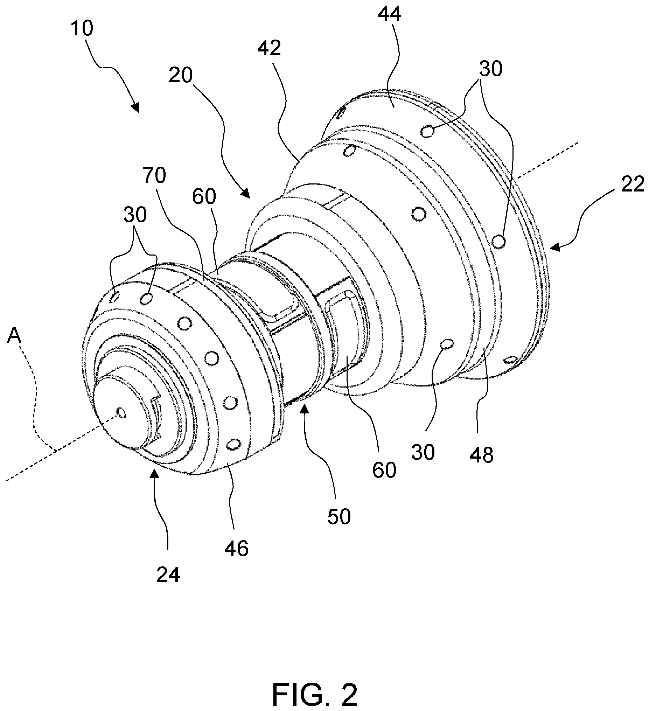

figure 2 is a perspective view showing the tracking assembly;

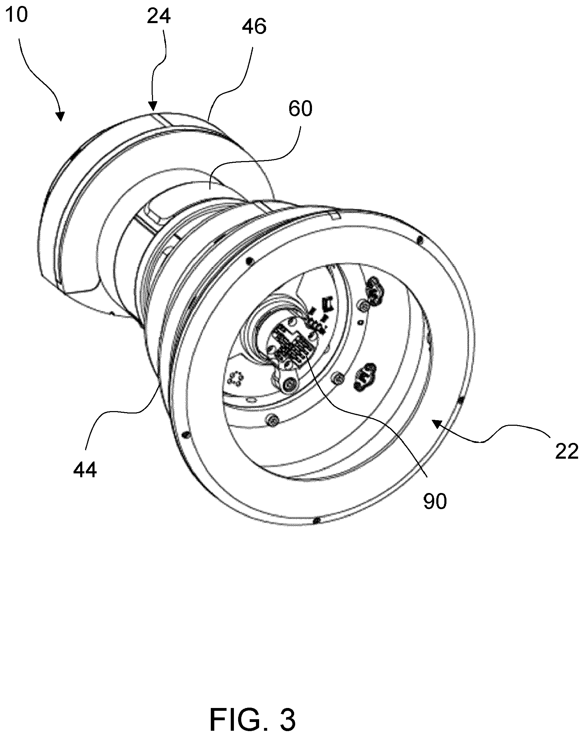

figure 3 is a perspective view showing the tracking assembly, seen from its proximal end where the tracking assembly is coupled to the robotic arm;

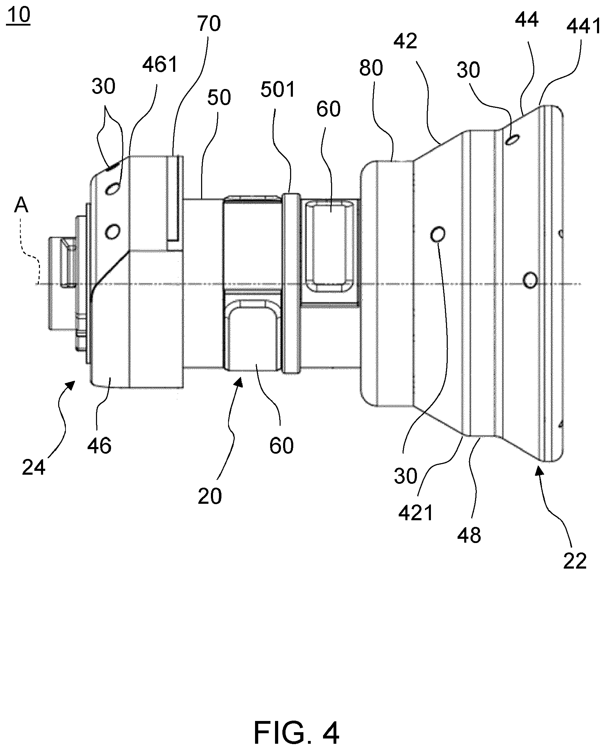

figure 4 is a side view showing the tracking assembly; and
figure 5 is a sectional view showing connection between the tracking assembly and the robotic arm.

### DETAILED DESCRIPTION OF EMBODIMENTS

Subject-matter disclosed herein relates to means for assisting surgical interventions to be performed onto a patient's body, including but not limited to, implantation of orthopedic implants such as pedicular screws in the spine, implantation of various orthopedic implants in bones, reduction and fixation of fractures during traumatological procedures, or positioning guides or canulae at a desired position with respect to a surgical target.

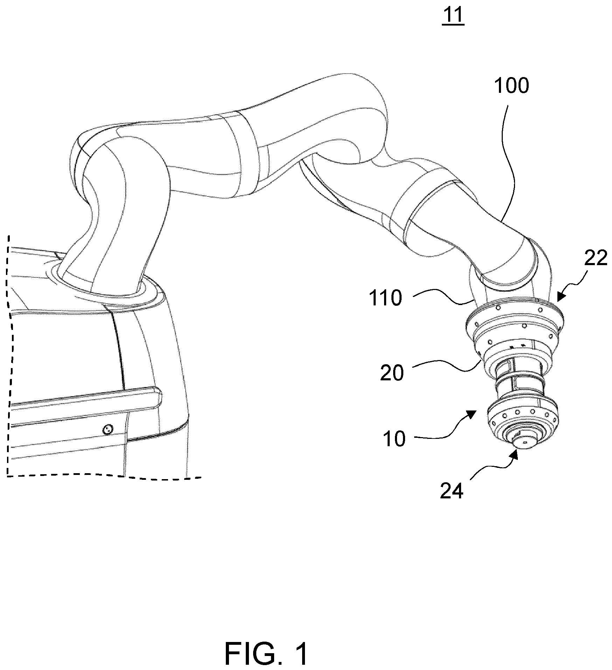

According to one embodiment, an assembly 10 for tracking a position and orientation of a robotic arm 100 is provided. The assembly 10 comprises a support body 20 extending along a central axis A. The support body 20 has a proximal end 22 and a distal end 24 opposite to the proximal end 22. The assembly 10 is configured to be attached to the robotic arm 100 at the proximal end 22 (see figure 1). The assembly 10 is configured to be attached to an end-effector such as a tool holder or a tool guide at the distal end 22.

The support body 20 comprises a hand grip 50 extending around the central axis A. The hand grip 50 is disposed between the proximal end 22 and the distal end 24. The hand grip 50 is configured to provide a grip to a user, typically a surgeon, for controlling the robotic arm 100. The hand grip 50 may have an ergonomic design to ensure a firm grip by the user.

The support body 20 comprises at least one first exterior portion 42 and 44 extending around the central axis A. In one embodiment, the at least one first exterior portion 42 and 44 may be disposed at the proximal end 22. In another embodiment, the at least one first exterior portion may be disposed between the hand grip 50 and the proximal end 22. In one embodiment, the first exterior portion 42 and 44 may have a generally cylindrical shape.

The support body 20 comprises at least one second exterior portion 46 extending around the central axis A. In one embodiment, the second exterior portion 46 may be disposed at the distal end 24. In another embodiment, the second exterior portion may be disposed between the distal end 24 and the hand grip 50. In one embodiment, the second exterior portion 46 may have a generally cylindrical shape.

The assembly 10 comprises at least three optical markers 30 disposed on the support body 20 and distributed around the central axis A. At least one optical marker 30 is provided on each first exterior portion 42 and 44 and on each second exterior portion 46. In one embodiment, two or more optical markers 30 may be provided on the first exterior portion 42 and 44 and/or on the second exterior portion 46. The respective optical markers 30 may be located at different positions around the central axis A so as to be spaced apart from each other axially and circumferentially.

In one embodiment, the optical markers 30 may be arranged to optimize localization of the robotic arm 100 and the corresponding end-effector, irrespective of an orientation of the robotic arm 100.

In one embodiment, a group of optical markers may be formed from three or more optical markers 30 distributed around the central axis A and respectively provided on the first exterior portions 42 and 44 and the second exterior portion 46. One or more of such groups of optical markers may be provided on the support body 20. Each group of the optical markers enables localization of the robotic arm 100 and the end-effector independently of each other, preferably irrespective of the position and orientation of the robotic arm 100. Yet each group of the optical markers may have different configurations from each other, in terms of the number of optical markers and/or arrangement of the optical markers 30 on the support body 20.

In one embodiment, the support body 20 may comprise two first exterior portions 42 and 44 spaced apart from each other along the central axis A and one second exterior portion 46. In one embodiment, the two first exterior portions 42 and 44 may be separated by a cylindrical portion 48 where no optical marker 30 is provided.

The first exterior portions 42 and 44 have an outermost edge 421 and 441 which lies farther from the central axis A in a direction perpendicular to the central axis A (or in a radial direction away from the central axis A) than an outermost edge 501 of the hand grip 50. Optionally, an outermost edge 461 of the second exterior portion 46 may also lie farther from the central axis A in the radial direction than the outermost edge 501 of the hand grip 50.

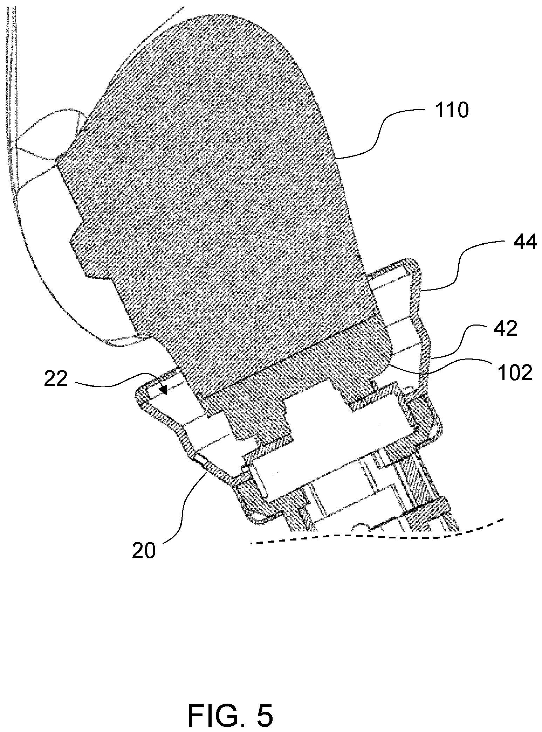

In use, the assembly 10 is attached to a wrist 110 of the robotic arm 100 via a distal flange 102. See figures 1 and 5.

A variety of end effectors, including but not being limited to, a tool holder or a tool guide (not shown), can be attached to the distal end 24 of the assembly 10. The robotic system 11 may be provided with a sensor, such as a camera, configured to detect the optical markers 30 provided on the assembly 10 as reference points. The surgical target may also be provided with a similar optical marker(s) in order to be detectable by the sensor. Such optical markers may also be provided at one or more points near the surgical target.

The end-effector may comprise a passive or active tool holder or guide. A passive tool holder may be configured to hold a tube in which the surgical tool is slidable. An active tool holder may be configured to hold an active surgical tool, which may be a drill, an ultrasonic cutter, a surgical bur, or any other active tools. The end-effector may also be provided with one or more optical markers to be detected by the sensor of the robotic system 11.

Based on the detected reference points where the optical markers 30 are provided, the robotic system 11 produces real-time data regarding the positions of the assembly 10 and the surgical tool relative to the robotic arm 100 and/or the surgical target.

According to the tracking assembly 10 as described above, the hand grip 50 is disposed between the first exterior portions 42 and 44 and second exterior portion 46. In addition, the optical markers 30 are provided not only at or near the proximal end 22 of the assembly 10, but also at or near the distal end 24. Therefore, the optical markers 30 or the detectable reference points are distributed over a relatively large area along the central axis A, substantially over the entire length of the assembly 10 along the central axis A. Due to the widely distributed optical markers 30, it is possible to improve accuracy in detecting the optical markers 30 in order to determine the position and the orientation of the assembly 10. Accordingly, the robotic system 11 can provide precise localization of the assembly 10 and therefore the surgical tool attached to the assembly 10.

The above-described advantage can be achieved without extending the axial length of the assembly 10, or without adding any extension in order to achieve wider distribution of the optical markers 30, thereby minimizing the distance between the surgical tool and the wrist 110 of the robotic arm 100. Therefore, precise localization of the surgical tool is possible while not affecting manipulability and reachability of the surgical tool for the user.

According to the disclosed configuration, at least some of the optical markers 30 are disposed at or near the proximal end 22 of the assembly 10. During surgical interventions, a space near the distal end 24 of the assembly 10 is usually surrounded by equipment or tools relevant to the surgery to be carried out. Thus, the optical markers 30 disposed at the proximal end 22 of the assembly 10 can contribute to optimization of the space available for the surgery, enhancing operability of the robotic system 11, improving visibility of the surgical area. In addition, the risk of collision with a patient's body or the surroundings can also be reduced.

According to one aspect of the present disclosure, more than one group of the optical markers may be provided in order to provide a redundant design. Since each group of the optical markers enables precise localization of the robotic arm 100 independently, the tracking of the robotic arm 100 and the end-effector is not interrupted even if an object such as a body fluid blocks a view of the sensor toward one of the optical markers.

According to the disclosed configuration, the hand grip 50 is smaller in size in the radial direction away from the central axis A than the exterior portions 42 and 44 where the optical markers 30 are provided. Thus, the risk of blocking a view of the sensor for detecting the optical markers 30 due to a hand of the user put on the hand grip 50 is reduced.

According to one embodiment, the support body 20 of the assembly 10 may be a monolithic piece. The monolithic support body 20 is advantageous since the positions of the optical markers 30 relative to each other remain unchanged after each use. Once the positional relationship is set up, for example, at the factory, no further recalibration is needed.

In another embodiment, the support body 20 may be made from two or more pieces. In this case, the position of the optical markers 30 relative to each other should be identified every time those pieces are taken apart, in order to ensure that localization of the surgical tool is accurate.

According to one embodiment, the assembly 10 may comprise at least one operational button 60 for controlling the robotic arm 100. The at least one operational button 60 may be provided on the hand grip 50 of the support body 20.

By providing the operation button 60 on the hand grip 50 which is relatively small in size in respect of the radial direction away from the central axis A, the user can operate the operation button 60 without affecting the tracking of the position and the orientation of the surgical tool based on the localization through the optical markers 30.

In one embodiment, two or more operation buttons 60 may be provided, circumferentially spaced apart from each other, for example by 90°, around the central axis A. By providing a space between the respective operation buttons 60, mishandling of the operation buttons 60 can be avoided.

In another embodiment, two pairs of the operation buttons 60 may be provided, each pair of the operation buttons 60 being provided away from each other along the central axis A. By providing the two pairs of the operation buttons 60, several operation modes can be selectively activated. Those operation modes may include but not be limited to, a hand guiding mode in which the robotic arm 100 is freely movable by the user, a computed trajectory mode in which the robotic arm 100 moves to a target position according to a computed trajectory, and a servo-controlled mode in which the robotic arm 100 autonomously moves in order to maintain the distal end 24 of the assembly 10 relative to the surgical target.

In one embodiment, the operational buttons 60 may be provided with different textures or a concave or convex form or any other form that helps the user distinguish one from another. In one embodiment, in order to provide additional security, the robotic system 10 may require the two buttons to be pressed simultaneously or additional operation means such as a foot pedal to be pressed simultaneously, in order to activate one of the operation modes or shift from one operation mode to another.

In one embodiment, the optical markers 30 may be light emitting elements. In one embodiment, the optical markers 30 may be infrared transmitters such as LEDs (light emitting diodes). In an alternative embodiment, the optical markers 30 may be passive optical markers, for example, refractive objects having a substantially spherical or spheroidal shape.

According to one embodiment, the robotic system 11 comprises a sterile cover (not shown) enclosing the robotic arm 100 and the support body 20, respectively. The sterile cover is configured to fit the first and second exterior portions 42, 44 and 46 of the support body 20. The sterile cover serves as protection for the assembly 10 from body fluids, pathogens or any other possible source of contamination before, during and after surgical interventions. In one embodiment, magnets or straps may be used to enhance fitting of the sterile cover onto the assembly 10.

In one embodiment, the sterile cover may be formed from a very thin film having a thickness of tens of micrometers. In one embodiment, the cover may be made of TPU (thermoplastic polyurethane). The very thin TPU cover is advantageous because it has very little refractive effect and therefore does not affect precise localization of the assembly 10 and the surgical tool attached thereto. In one embodiment, the cover may be made by welding two or more parts together. In that case the welding line should be placed at a portion other than onto the optical markers 30 in order to avoid affecting precise localization of the surgical tool.

According to one embodiment, at least one of the first and second exterior portions 42, 44 and 46 may be formed to taper toward the distal end 24. In one embodiment, at least one of the first and second exterior portions 42, 44 and 46 may have a conical shape having a decreasing diameter toward the distal end 24. This configuration allows the sterile cover to be fitted onto the assembly 10 from the distal end 24 toward the proximal end 22, like a glove, while maintaining close fitting of the cover onto the assembly 10.

In the embodiment where the exterior portions 42, 44 and 46 and the hand grip 50 have a generally cylindrical shape, the fitting of the sterile cover onto the assembly 10 can be advantageously facilitated.

According to one embodiment, each of the first exterior portions 42 and 44 has an outermost edge at an equal distant or greater distance from the central axis A in a direction perpendicular to the central axis than the outermost edge 461 of the second exterior portion 46. This configuration prevents the sterile cover from being constrained by the second exterior portion 46, thereby facilitating fitting of the sterile cover onto the assembly 10.

According to one embodiment, each optical marker 30 may be provided in a cavity formed on the corresponding exterior portion 42, 44 and 46 in such a way that the optical marker 30 is substantially flush with a surface of the corresponding exterior portion 42, 44 and 46. This configuration ensures that no protrusion is formed on the exterior surface of the assembly 10, providing no constraint during the process of fitting the sterile cover onto the assembly 10, also enabling close fitting of the cover onto the assembly 10.

According to the disclosed configuration, the hand grip 50 of the assembly 10 is relatively small in size with respect to the radial direction away from the central axis A. The smaller hand grip 50 disposed between the first and second exterior portions 42, 44 and 46 can relieve tension while the sterile cover is fitted onto the assembly 10, thereby facilitating the fitting process of the sterile cover.

According to one embodiment, the proximal end 22 of the support body 20 may be attached to the robotic arm 100 in such a way that the distal flange 102 of the robotic arm 100 lies within an interior of the proximal end 22. See figure 5. This configuration contributes to reducing the distance between the distal end 24 of the assembly 10 and the wrist 110 of the robotic arm 100.

According to one embodiment, the assembly 10 may comprise a visible light emitting ring 70 extending around the central axis A. The light emitting ring 70 serve as an indicator by which the user becomes aware which operation mode is currently in use. In one embodiment, the light emitting ring 70 may comprise a plurality of LEDs configured to emit light of predetermined colors and/or in predetermined blinking patterns, depending on the operation mode selected. The light emitting ring 70 may be provided on the hand grip 50 or near the hand grip 50, substantially axially distant from the optical markers 30.

According to one embodiment, the assembly 10 may comprise a storage device (not shown) for storing data for tracking the position and orientation of the robotic arm 100. In one embodiment, the data stored in the storage device may be calibration data of the optical markers 30, including but not being limited to, data associating the patterns of the optical markers 30 with the position and the orientation of the assembly 10.

According to one embodiment, the assembly 10 may comprise a communication module for transmitting the calibration data to a control unit of the robotic system 11.

According to one embodiment, the robotic system 11 may comprise a monitor display (not shown) for displaying information regarding the position of the surgical tool relative to the surgical target or the current operation mode of the robotic arm 100.

According to one embodiment, the assembly 10 may comprise at least one electrical connector 90 arranged in an interior of the support body 20, the at least one electrical connector 90 being configured to engage with a corresponding electrical connector of the robotic arm 100. According to one embodiment, the electrical connector 90 may be electrically connected to the at least one operational button 60, the light emitting elements of the optical markers 30, the visible light emitting ring 70 and the storage device. By providing the electric connectors in the assembly 10 where the assembly 10 is coupled to the robotic arm 100, the mechanical connection between the two can also result in required electrical connection for transmission of electric signals or power supply, thereby facilitating the setting up process of the robotic system 11.

In one embodiment, the proximal end 22 of the assembly 10 may be coupled to the robotic arm 100 by means of a pogo-pin connector. With this configuration, the mechanical connection as well as the electrical connection between the assembly 10 and the robotic arm 100 can be easily established.

The detailed explanations are provided by way of example but not intended to limit the scope of invention. Rather, the scope of the invention is defined by the appended claims.

## Claims

1. An assembly (10) for tracking a position and orientation of a robotic arm (100), the assembly comprising:
a support body (20) extending along a central axis (A) and having a proximal end (22) and a distal end (24) opposite to the proximal end, the proximal end being configured to be attached to the robotic arm (100), the distal end (24) being configured to be attached to a tool holder or a tool guide, the support body (20) further comprising a hand grip (50) extending around the central axis and configured to provide a grip to a user for controlling the robotic arm; and
at least three optical markers (30) disposed on the support body and distributed around the central axis,
**characterized in that**
the hand grip is disposed between the proximal end and the distal end, **in that** the support body further comprises:
at least one first exterior portion (42, 44) extending around the central axis and being disposed at the proximal end or between the hand grip and the proximal end, each first exterior portion being provided with at least one of the optical markers (30); and
at least one second exterior portion (46) extending around the central axis and disposed at the distal end or between the distal end and the hand grip, each second exterior portion being provided with at least one of the optical markers (30), and **in that**
the first exterior portion has an outermost edge (421, 441) which lies farther from the central axis in a direction perpendicular to the central axis than an outermost edge (501) of the hand grip.

2. The assembly (10) according to claim 1, wherein the support body (20) is a monolithic piece.

3. The assembly (10) according to claim 1 or 2, further comprising at least one operational button (60) for controlling the robotic arm, the at least one operational button being provided on the hand grip (50) of the support body (20).

4. The assembly (10) according to any one of claims 1 to 3, wherein at least one of the first and second exterior portions (42, 44, 46) tapers toward the distal end.

5. The assembly (10) according to any one of claims 1 to 4, wherein each of the first exterior portions (42, 44) has an outermost edge at an equal distant or greater distance from the central axis (A) in a direction perpendicular to the central axis than the outermost edge (461) of the second exterior portion (46).

6. The assembly (10) according to any one of claims 1 to 5, wherein the support body comprises two of the first exterior portions (42, 44) spaced apart from each other along the central axis and one of the second exterior portion (46).

7. The assembly (10) according to any one of claims 1 to 6, wherein each optical marker (30) is provided in a cavity formed on the corresponding exterior portion (42, 44, 46) in such a way that the optical marker is substantially flush with a surface of the corresponding exterior portion (42, 44, 46).

8. The assembly (10) according to any one of claims 1 to 7, wherein the optical markers (30) are light emitting elements.

9. The assembly according to any one of claims 1 to 8, further comprising a visible light emitting ring (70) extending around the central axis.

10. The assembly according to any one of claims 1 to 9, further comprising a storage device for storing data for tracking the position and orientation of the robotic arm.

11. The assembly according to any one of claims 1 to 10, further comprising at least one electrical connector (90) arranged in an interior of the support body, the at least one electrical connector being configured to engage with a corresponding electrical connector of the robotic arm (100).

12. The assembly according to claim 11 in combination with at least one of claims 3, 8, 9 and 10, wherein the electrical connector is electrically connected to the at least one operational button (60), the light emitting elements, the visible light emitting ring (70) and the storage device.

13. A robotic system (11) comprising:
a multiple joints robotic arm (100); and
the assembly (10) according to any one of claims 1 to 12 attached to a distal flange (102) of the robotic arm.

14. The robotic system according to claim 13, wherein the proximal end (22) of the support body (20) is attached to the robotic arm in such a way that the distal flange of the robotic arm lies within an interior of the proximal end.

15. The robotic system according to claim 13 or 14, further comprising a sterile cover enclosing the robotic arm (100) and the support body (20), wherein the sterile cover fits the first and second exterior portions (42, 44, 46) of the support body.
